# EUROPEAN PATENT APPLICATION

(11) **EP 4 574 964 A1**
(43) Date of publication of application: **25.06.2025**
(21) Application number: 23855195.6
(22) Date of filing: 18.08.2023
(51) Int. Cl.: C12N 1/20, A01N 63/20, A61K 35/744, A61K 35/745, A23L 33/135, A61P 31/04, A61P 19/10, A61P 25/24, C12R 1/46, C12R 1/01

(54) **NOVEL LACTIC ACID BACTERIA AND USES THEREOF**

(30) Priority: 18.08.2022 KR 20220103472
(71) Applicant: Dong Wha Pharm. Co., Ltd., Seoul 04522 (KR)
(72) Inventor: KIM, Dong Hyun, Seoul 02823 (KR)
(74) Representative: Stolmár & Partner Patentanwälte PartG mbB
(86) International application number: PCT/KR2023/012291
(87) International publication number: WO 2024/039229

(57) **Abstract**

The present invention relates to novel lactic acid bacteria, *Lactococcus lactis* P32 (KCCM13222P), *Bifidobacterium bifidum* P45 (KCCM13223P), or a mixture thereof, and uses thereof. Strains and a mixture thereof, according to the present invention, can be effectively used in the prevention or treatment of an inflammatory disease, osteoporosis, depressive disorder, or anxiety disorder.

## Description

### TECHNICAL FIELD

The present invention relates to novel lactic acid bacteria, *Lactococcus lactis* P32 (KCCM13222P) and *Bifidobacterium bifidum* P45 (KCCM13223P), or uses thereof.

### BACKGROUND ART

Under normal conditions, the vagina of a woman is in an environment of weak acidity due to many lactic acid bacteria inside the vagina, and this environment serves to suppress the invasion of pathogenic microorganisms or the proliferation of fungi. Such an environment of weak acidity in the vagina may be broken by external factors, for example, excessive vaginal cleansing, long-term administration of an antibiotic or a contraceptive, and disruption of hormone balance due to pregnancy, childbirth, menopause, or the like in a case where there is a change in the quantity of lactic acid bacteria, which are beneficial bacteria that are normally present in the vagina. This is known to be the cause of vaginitis, which causes secretions, itching, and pain.

Many methods for preventing and treating such vaginitis have been studied extensively. Recently, as a treatment method for a bacterial infection of the vagina, a method, in which a therapeutic agent for vaginitis such as metronidazole is orally administered or administered in a cream form, is generally used. However, the use of broad-spectrum antibiotics such as metronidazole is evaluated to be undesirable because it not only entails the problem of antibiotic resistance but also may kill a wide range of normal bacteria in the vagina, including beneficial lactic acid bacteria. In addition, it has been also reported that the long-term use of antibiotics may cause systemic toxicity due to the absorption of antibiotics through the vagina. As a result, it is urgent to develop a composition for prevention or treatment of vaginitis, which can prevent and/or treat vaginal infections and is safe and thus side effects are not problematic.

On the other hand, osteoporosis is a complex disease that is also affected by genetic factors or environmental factors such as diet and lifestyle, and particularly in women, bone decrease rapidly proceeds by hormonal changes in a case where menopause is reached. Currently, the therapeutic agent for osteoporosis which is commercially available includes vitamin D, a female hormone drug, a bisphosphonate formulation, a selective estrogen receptor regulator, a calcitonin formulation, and the like, mostly of which depends on the method in which the activity of osteoclasts are reduced to regulated bone absorption, thereby preventing bone loss. However, complete curing is limited since the therapies for osteoporosis through the suppression of activation of osteoclasts are not therapies that can fundamentally treat osteoporosis, and thus there is an essential requirement for an increase in the activity of osteoblasts, which makes it possible to achieve an increase in actual bone density and bone reinforcement.

Most therapeutic agents for osteoporosis that are currently used are estrogen-based substances, which have a problem in that side effects such as cancer, cholelithiasis, and thrombosis are exhibited in a case of being subjected to long-term administration. Osteoporosis is a disease that cannot be cured by only short-term administration of drugs and essentially requires long-term administration of drugs. Therefore, it is required to develop a new substance that has no side effects described above-described even in a case where a drug is subjected to long-term administration and has a medicinal efficacy excellent enough to replace estrogen.

On the other hand, an infectious disease, a hormone imbalance (menopausal) disease, and the like may be accompanied by diseases such as depression and anxiety disorder. The use of drugs such as antidepressants that are used to treat these mental diseases is limited since severe side effects such as a cardiovascular disease and suicide may exhibited, and these mental diseases are diseases that cannot be treated by only short-term administration of drugs and thus essentially require long-term administration. Therefore, it is required to development a new substance that has an excellent medicinal efficacy without having side effects.

### DISCLOSURE OF THE INVENTION

### TECHNICAL PROBLEM

The object of the present invention is to provide a strain of *Lactococcus lactis* P32 KCCM13222P.

Another object of the present invention is to provide a strain of *Bifidobacterium bifidum* P45 KCCM13223P.

Another object of the present invention is to provide an antimicrobial composition that comprises *Lactococcus lactis* P32 KCCM13222P, *Bifidobacterium bifidum* P45 KCCM13223P, or a mixture thereof.

Another object of the present invention is to provide a pharmaceutical composition that comprises *Lactococcus lactis* P32 KCCM13222P, *Bifidobacterium bifidum* P45 KCCM13223P, or a mixture thereof.

Another object of the present invention is to provide a food composition that comprises *Lactococcus lactis* P32 KCCM13222P, *Bifidobacterium bifidum* P45 KCCM13223P, or a mixture thereof.

Another object of the present invention is to provide a method for prevention or treatment of an inflammatory disease, osteoporosis, depressive disorder, or anxiety disorder, the method including a step of administering, to an individual, *Lactococcus lactis* P32 KCCM13222P, *Bifidobacterium bifidum* P45 KCCM13223P, or a mixture thereof.

### TECHNICAL SOLUTION

An aspect of the present invention, which aims to achieve the objects described above, relates to *Lactococcus lactis* P32 KCCM13222P.

The *Lactococcus lactis* P32 according to the present invention is characterized by being a novel lactic acid bacterium, which is isolated and identified from the feces of a healthy human.

The base sequence of 16S rDNA for identification and classification of *Lactococcus lactis* P32 according to the present invention is the same as SEQ ID NO. 1 attached to the present specification. As a result, The *Lactococcus lactis* P32 according to the present invention may comprise 16S rDNA of SEQ ID NO. 1.

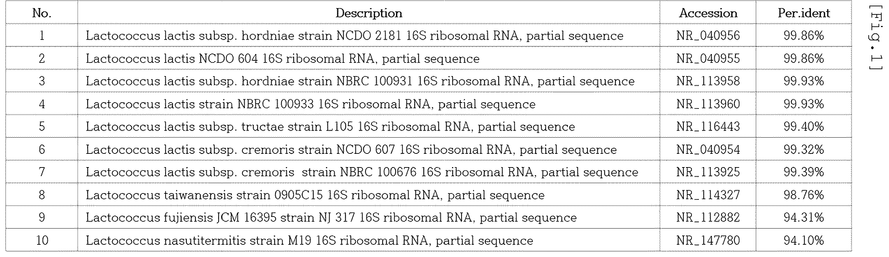

As a result of analyzing the base sequence of 16S rDNA, which is set forth in SEQ ID NO. 1, a 99% of homology with the publicly known strains of *Lactococcus lactis,* and the closest taxonomic relationship with the *Lactococcus lactis* was exhibited in terms of the molecular phylogeny (Fig. 1). Therefore, the lactic acid bacterium was identified as *Lactococcus lactis,* named as *Lactococcus lactis* P32, and deposited to the Korean Culture Center of Microorganisms at 2022.08.03 (KCCM13222P).

The *Lactococcus lactis* P32 according to the present invention is a Gram-positive micrococcus. More specific physiological characteristics of *Lactococcus lactis* P32 can be analyzed according to the conventional methods in the related technical field, and specifically, Lactococcus lactis P32 can use, as carbonic sources, D-ribose, D-xylose, D-galactose, D-glucose, D-fructose, D-mannose, mannitol, N-acetyl-glucosamine, amygdalin, arbutin, esculin, salicin, cellobiose, maltose, lactose, sucrose, trehalose, starch, and gentiobiose.

Another aspect of the present invention relates to *Bifidobacterium bifidum* P45 KCCM13223P.

The *Bifidobacterium bifidum* P45 according to the present invention is characterized by being a novel lactic acid bacterium, which is isolated and identified from the feces of a healthy human.

The base sequence of 16S rDNA for identification and classification of the *Bifidobacterium bifidum* P45 according to the present invention is the same as SEQ ID NO. 2 attached to the present specification. As a result, the Bifidobacterium bifidum P45 according to the present invention may contain 16S rDNA of SEQ ID NO. 2.

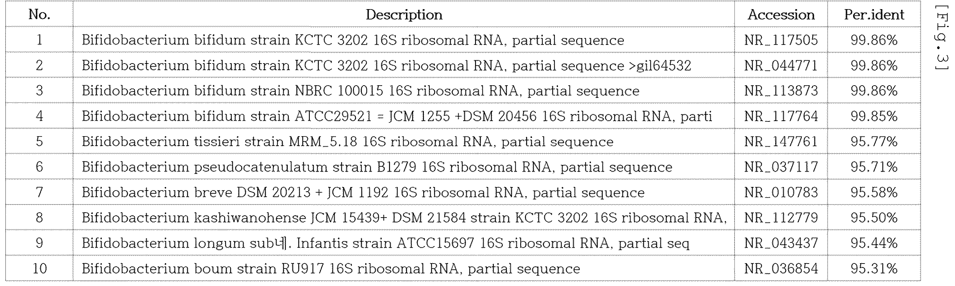

As a result of analyzing the base sequence of 16S rDNA, which is set forth in SEQ ID NO. 2, a 99% of homology with the publicly known strains of *Bifidobacterium bifidum,* and the closest taxonomic relationship with the *Bifidobacterium bifidum* was exhibited in terms of the molecular phylogeny(Fig. 3). Therefore, the lactic acid bacterium was identified as *Bifidobacterium bifidum,* named as *Bifidobacterium bifidum* P45, and deposited to the Korean Culture Center of Microorganisms at 2022.08.03 (KCCM13223P).

The *Bifidobacterium bifidum* P45 according to the present invention is a Gram-positive bacillus. More specific physiological characteristics of *Bifidobacterium bifidum* P45 can be analyzed according to the conventional methods in the related technical field, and specifically, *Bifidobacterium bifidum* P45 can use, as carbonic sources, D-glucose, D-lactose, D-saccharose, D-maltose, L-arabinose, gelatin, eschulin sulfuric acid, D-cellobiose, D-mannose, and D-raffinose.

Another aspect of the present invention relates to an antimicrobial composition containing *Lactococcus lactis* P32 KCCM13222P, *Bifidobacterium bifidum* P45 KCCM13223P, or a mixture thereof.

In addition, another aspect of the present invention relates to a pharmaceutical composition for prevention or treatment of infection with *Gardnerella vaginalis* or *Staphylococcus aureus,* the pharmaceutical composition containing the antimicrobial composition.

In one exemplary embodiment according to the present invention, since the *Lactococcus lactis* P32 and/or the *Bifidobacterium bifidum* P45 has been confirmed to suppress infection with *Gardnerella vaginalis* and/or *Staphylococcus aureus* (Table 6), the *Lactococcus lactis* P32 and/or the *Bifidobacterium bifidum* P45 may be used for the antimicrobial use and furthermore, they may be used for use for prevention or treatment for infection with *Gardnerella vaginalis* or *Staphylococcus aureus.*

Another aspect of the present invention relates to a pharmaceutical composition for prevention or treatment of an inflammatory disease or osteoporosis, the pharmaceutical composition that comprises *Lactococcus lactis* P32 KCCM13222P, *Bifidobacterium bifidum* P45 KCCM13223P, or a mixture thereof.

In the present invention, the "inflammatory disease" collectively refers to diseases in which inflammation is the main lesion. The inflammatory disease according to the present invention may be any one or more selected from the group including arthritis, gout, hepatitis, obesity, keratitis, gastritis, enteritis, nephritis, colitis, diabetes, tuberculosis, bronchitis, pleurisy, peritonitis, spondylitis, pancreatitis, inflammatory pain, urethritis, cystitis, vaginitis, arteriosclerosis, septicemia, and periodontitis. More specifically, the above-described inflammatory disease may be vaginitis; however, it is not limited thereto.

The vaginitis is an inflammatory disease that occurs in the vagina of a woman, and the causes thereof are largely divided into three main causes, which are *Bacterial vaginosis* (BV), *Candida vaginitis* (CV), and *Trichomonas vaginalis.* Among these, bacterial vaginosis has been recognized as the most common vaginitis in recent years, and it has been reported that *Atopobium vaginae, Gardnerella vaginalis, Prevotella bivia,* or the like has been found in vaginal secretions isolated from patients with bacterial vaginosis. In particular, *Gardnerella vaginalis* is known to be a main causative bacterium of vaginitis. The novel lactic acid bacteria according to the present invention exhibits a suppressive effect effective on *Gardnerella vaginalis* and thus are effective in the treatment of vaginitis.

In one exemplary embodiment according to the present invention, the *Lactococcus lactis* P32 and/or the *Bifidobacterium bifidum* P45 has been confirmed to reduce the expression of inflammatory cytokines (Table 6), and thus the *Lactococcus lactis* P32 and/or the *Bifidobacterium bifidum* P45 can be used for use for treatment of an inflammatory disease.

In the present invention, the "osteoporosis" is a disease caused by hyperactivation of absorption as compared with bone formation, which is caused by an unbalance between osteoblasts and osteoblasts, since the compact substance of the bone becomes thin due to the reduction of calcium in the bone tissue, the marrow cavity is consequently widened, and the bone is weakened as the symptom progresses, bone fracture is likely to occur even by a small shock. The causes of osteoporosis are known to be aging, lack of exercise, low body weight, smoking, low calcium diet, menopause, ovarian excision, and the like. Particularly in women, bone decrease continuously proceeds after the age of 30 years, and bone decrease rapidly proceeds by hormonal changes in a case where menopause is reached.

In one exemplary embodiment according to the present invention, the *Lactococcus lactis* P32 and/or the *Bifidobacterium bifidum* P45 has been confirmed to exhibit an amelioration effect on osteoporosis indicators (Table 9 and Table 10).

Another aspect of the present invention relates to a pharmaceutical composition for prevention or treatment of depressive disorder or anxiety disorder, where the pharmaceutical composition comprises *Lactococcus lactis* P32 KCCM13222P, *Bifidobacterium bifidum* P45 KCCM13223P, or a mixture thereof.

The "depressive disorder" is a disease that accompanies decreased desire and depression as major symptoms and causes a variety of cognitive and mental-physical symptoms, thereby bringing the reduction of the daily function, and the depressive disorder may be any one or more selected from the group including major depressive disorder, persistent depressive disorder, dysthymia, disruptive mood dysregulation disorder, premenstrual dysphoric disorder, substance/medication-induced depressive disorder induced by a drug, depressive disorder due to another medical condition, other specified depressive disorders, and unspecified depressive disorder; however, the depressive disorder is not limited thereto.

The "anxiety disorder" is a mental disorder that disrupts everyday life due to a variety of forms of abnormal, pathological anxiety and fear, and the anxiety disorder may be any one or more selected from the group including generalized anxiety disorder, specific phobia, agoraphobia, social anxiety disorder, panic disorder, separation anxiety, and selective mutism; however, the anxiety disorder is not limited thereto.

The "depressive disorder" or the "anxiety disorder" may be caused by an inflammatory disease or a female hormone imbalance, which is not limited thereto.

In the exemplary embodiment according to the present invention, the fact that the *Lactococcus lactis* P32 and/or the *Bifidobacterium bifidum* P45 markedly ameliorates the depression and anxiety behavior in a mouse model in which the female hormone imbalance was caused by inflammation induction and/or ovariectomy has been confirmed by carrying out a behavioral experiment and measuring the numerical values of BDNF and Claudin -5 (Table 8 to Table 10).

Specifically, the above-described mixture in the above-described pharmaceutical composition may be obtained by mixing *Lactococcus lactis* P32 KCCM13222P and *Bifidobacterium bifidum* P45 KCCM13223P at a ratio of 1:1 to 4:1 in terms of colony forming unit (CFU); however, the above-described mixture is not limited thereto.

In the exemplary embodiment according to the present invention, a mice model for vaginitis and/or a mice model for osteoporosis disease was treated with *Lactococcus lactis* P32 and *Bifidobacterium bifidum* P45 and then it was confirmed that the effect of prevention or treatment of osteoporosis is excellent (Table 8 to Table 10).

More specifically, each of the *Lactococcus lactis* P32 KCCM13222P and the *Bifidobacterium bifidum* P45 KCCM13223P may be a live bacterium thereof, an inactivated bacterium thereof, a culture product thereof, a disrupted product thereof, an extract thereof; however, the *Lactococcus lactis* P32 KCCM13222P or the *Bifidobacterium bifidum* P45 KCCM13223P can be applied without limitation as long as it having a form that is capable of achieving the intended effect.

In the present invention, the "live bacterium" means the novel lactic acid bacterium itself, according to the present invention, the "inactivated bacterium" means a lactic acid bacterium that has been subjected to a heating, pressurization, or drug treatment, and the "disrupted product" means lactic acid bacteria destroyed by an enzyme treatment, homogenization, or ultrasound treatment of lactic acid bacteria.

In the present invention, the "extract" means a product obtained by subjecting lactic acid bacteria to extraction with a publicly known extraction solvent.

In the present invention, the "culture product" or the "culture solution" means a product obtained by culturing lactic acid bacteria in a publicly known culture medium, and the product may contain novel lactic acid bacteria. The above-described culture medium may be selected from a liquid culture medium or a solid culture medium, and for example, it may be an MRS liquid culture medium, a GAM liquid culture medium, an MRS agar culture medium, a GAM agar culture medium, or a BL agar culture medium; however, the culture medium is not limited thereto.

The pharmaceutical composition according to the present invention may be manufactured in a pharmaceutical formulation by using a method well known in the art so that the rapid, sustained, or delayed release of active ingredients can be provided after administration to mammals. In the manufacturing of the formulation, the pharmaceutical composition according to the present invention may additionally contain a pharmaceutically acceptable carrier to the extent that the activity of the novel lactic acid bacteria is not inhibited.

Another aspect of the present invention relates to a method for prevention or treatment of an inflammatory disease, osteoporosis, depressive disorder, or anxiety disorder, where the method includes a step of administering a pharmaceutical composition containing the above-described strain or a mixture of the above-described strains, to a subject.

The "inflammatory disease", the "osteoporosis", the "depressive disorder", and the "anxiety disorder" are the same as those described above, and specifically, the inflammatory disease may be vaginitis.

The above-described subject refers to an animal and typically, it may be a mammal that can exhibit a beneficial effect by treatment using the lactic acid bacteria according to the present invention. The desirable examples of such subjects may include a primate such as a human.

One aspect of the present invention relates to a food composition for prevention or amelioration of an inflammatory disease or osteoporosis, where the food composition comprises *Lactococcus lactis* P32 KCCM13222P, *Bifidobacterium bifidum* P45 KCCM13223P, or a mixture thereof.

The "inflammatory disease" and the "osteoporosis" are the same as those described. Specifically, the inflammatory disease may be vaginitis.

Another aspect of the present invention relates to a food composition for prevention or amelioration of depressive disorder or anxiety disorder, where the pharmaceutical composition comprises *Lactococcus lactis* P32 KCCM13222P, *Bifidobacterium bifidum* P45 KCCM13223P, or a mixture thereof.

The "depressive disorder" or the "anxiety disorder" may be caused by an inflammatory disease or a female hormone imbalance, which is not limited thereto.

Specifically, the mixture of the above-described strains in the above-described pharmaceutical composition may be obtained by mixing *Lactococcus lactis* P32 KCCM13222P and *Bifidobacterium bifidum* P45 KCCM13223P at a ratio of 1:1 to 4:1 in terms of colony forming unit (CFU); however, the above-described mixture is not limited thereto.

There are no specific restrictions on the kind of the food described above. The food to which lactic acid bacteria can be added includes sausages, meats, bread, chocolates, snacks, candies, confectionery, ramen, pizza, other noodles, chewing gums, dairy products including ice creams, various soups, beverages, tea, drinks, alcoholic beverages, vitamin complexes, and the like. The liquid ingredient to be added in addition to the novel lactic acid bacteria is limited to this in a case of carrying out formulation with drinking water, however, similar to the typical beverages, various flavoring agents or natural carbohydrates can be contained as additional ingredients. The natural carbohydrates described above may be a monosaccharide (for example, glucose or fructose), a disaccharide (for example, maltose or sucrose), and a polysaccharide (for example, a conventional sugar such as dextrin or cyclodextrin), as well as a sugar alcohol such as xylitol, sorbitol, or erythritol.

In addition, specifically, the lactic acid bacteria contained in the pharmaceutical composition according to the present invention may be a live bacterium thereof, an inactivated bacterium thereof, a culture thereof, a disrupted product thereof, an extract thereof; however, the lactic acid bacteria can be used without limitation as long as they have a form of lactic acid bacteria that are capable of achieving an effect of prevention or treatment of an inflammatory disease or osteoporosis. The "live bacterium", the "inactivated bacterium", the "culture product", the "disrupted product", and the "extract" are the same as those described above.

Specifically, the above-described food may be a healthy functional food. The healthy functional food is a food that emphasizes the bio-regulatory function of a food and is a food that is imparted with an additional value so that the food acts on and exhibits a specific purpose by using a physical, biochemical, or bioengineering method. The ingredients of such a healthy functional food are designed and processed to sufficiently exhibit functions of body regulation, which are involved in biophylaxis and body rhythm regulation, and disease prevention and recovery, and they may include a food auxiliary additive, a sweetener, or a functional raw material, which is acceptable as the food.

In a case where the *Lactococcus lactis* P32 or the *Bifidobacterium bifidum* P45 according to the present invention is used as a healthy functional food (or a healthy functional beverage additive), the above-described novel lactic acid bacteria may be added as they are or may be used together with other foods or food ingredients, the above-described novel lactic acid bacteria may be used appropriately according to the conventional method. The mixing amount of the *Lactococcus lactis* P32 and/or the *Bifidobacterium bifidum* P45 may be appropriately determined according to the purpose of use of the *Lactococcus lactis* P32 and/or the *Bifidobacterium bifidum* P45 (prevention, health, amelioration, or therapeutic treatment) .

Another aspect of the present invention relates to a method for prevention or treatment of an inflammatory disease, osteoporosis, depressive disorder, or anxiety disorder, the method including a step of administering, to an individual, *Lactococcus lactis* P32 KCCM13222P, *Bifidobacterium bifidum* P45 KCCM13223P, or a mixture thereof.

The "inflammatory disease", the "osteoporosis", the "depressive disorder", and the "anxiety disorder" are the same as those described above. Specifically, the inflammatory disease may be vaginitis.

The "individual" includes an animal or human having an inflammatory disease, osteoporosis, depressive disorder, or anxiety disorder, symptoms of which can be ameliorated by the administration of the *Lactococcus lactis* P32 KCCM13222P and the *Bifidobacterium bifidum* P45 KCCM13223P according to the present invention, or a mixture thereof. By administering the strains or strain mixture according to the present invention, or a composition containing these to an individual, it is possible to effectively prevent or treat an inflammatory disease, osteoporosis, depressive disorder, or anxiety disorder.

The "administration" means the introduction of a predetermined substance to a human or animal by any appropriate method, and the administration route of the strains or strain mixture according to the present invention, or a composition containing these may be such that the strains or strain mixture according to the present invention, or the composition containing these can be administered orally or parenterally through any general route as long as it is possible to reach the target tissue. In addition, the therapeutic composition according to the present invention may be administered by any device that makes it possible for the active ingredient to move to the target cells.

The desired administration dosage of the strains or strain mixture according to the present invention, or a composition containing these varies depending on the condition and body weight of the patient, the degree of the disease, the drug form, the route of administration and the duration; however, it may be appropriately selected by those skilled in the art.

Another aspect of the present invention relates to use of *Lactococcus lactis* P32 KCCM13222P, *Bifidobacterium bifidum* P45 KCCM13223P, or a mixture thereof, the use being for prevention or treatment of an inflammatory disease, osteoporosis, depressive disorder, or anxiety disorder.

The "inflammatory disease", the "osteoporosis", the "depressive disorder", and the "anxiety disorder" are the same as those described above. Specifically, the inflammatory disease may be vaginitis.

### ADVANTAGEOUS EFFECTS

The *Lactococcus lactis* P32 KCCM13222P and the *Bifidobacterium bifidum* P45 KCCM13223P according to the present invention, or a mixture thereof has an excellent amelioration effect on vaginitis, osteoporosis, depressive disorder, or anxiety disorder.

The effect of the present invention is not limited to the above-described effect and thus shall be understood to include all effects which can be inferred from the configuration of the present invention, which is described in detailed descriptions of the present invention or claims.

### BRIEF DESCRIPTION OF THE DRAWINGS

Fig. 1 shows the homology of 16S rDNA between the disclosed strain *Lactococcus lactis* P32 (KCCM13222P) and other *Lactococcus lactis* strains.

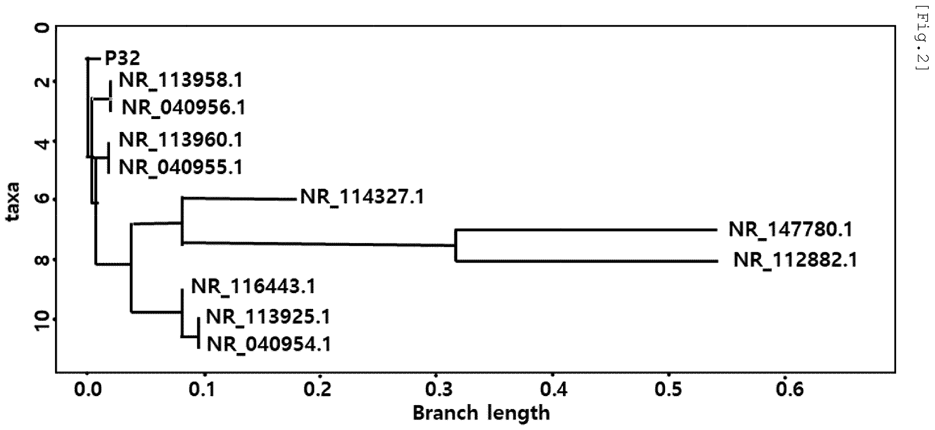

Fig. 2 shows the phylogenetic systematic characteristics of *Lactococcus lactis* P32 KCCM13222P.
Fig. 3 shows the homology of 16S rDNA between publicly known *Bifidobacterium bifidum* strains, and *Bifidobacterium bifidum* P45 (KCCM13223P) is analyzed.

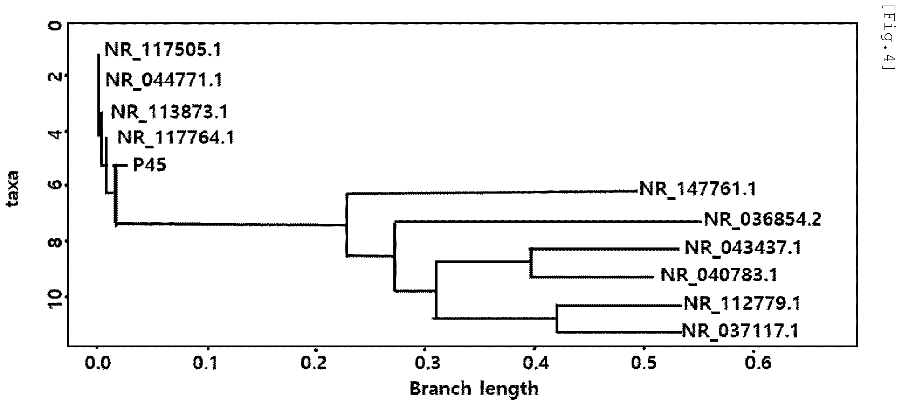

Fig. 4 shows the phylogenetic systematic characteristics of *Bifidobacterium bifidum* P45 (KCCM13223P).
Fig. 5 to Fig. 7 show the amelioration effects of the probiotic strain of this invention on vaginitis, as demonstrated in Experiments 6 to 8 of this invention.

### MODE FOR CARRYING OUT THE INVENTION

Hereinafter, the present invention will be described in detail according to examples. However, the following examples are only for the exemplary description of the present invention, and thus the present invention is not limited to the following examples.

### Example 1. Isolation and identification of lactic acid bacteria

### 1-1. Isolation of lactic acid bacteria from human feces

Feces of a healthy human were placed and suspended in a GAM liquid culture medium (GAM broth, Nissui Pharmaceutical, Japan). Subsequently, the supernatant was taken, transplanted in a GAM agar culture medium (GAM agar medium, Nissui Pharmaceutical, Japan), an MRS agar culture medium (BD, USA), or a BL agar culture medium (BL agar medium; Nissui Pharmaceutical, Japan), and cultured anaerobically at 37°C for approximately 48 hours, and then strains that formed colony were isolated.

### 1-2. Identification of isolated lactic acid bacteria

The strains isolated from human feces were subjected to the analysis of Gram staining, physiological characteristics, sequencing of the 16S rDNA gene, and the like, the strains were identified, and the strain names were assigned to the identified strains. The strain names of the assigned lactic acid bacteria are as shown in Table 1 below. Specifically, they are six species of *Lactobacillus plantarum* (numbers 1 to 6 in Table 1), five species of *Lactobacillus sakei* (numbers 6 to 10 in Table 1), two species of *Lactobacillus reuteri* (numbers 11 and 12 in Table 1), three species of *Lactobacillus rhamnosus* (numbers 13 to 15 in Table 1), five species of *Lactococcus lactis* (numbers 16 to 20 in Table 1), three species of *Bifidobacterium adolescentis* (numbers 21 to 23 in Table 1), three species of *Bifidobacterium bifidum* (numbers 24 to 26 in Table 1), and four species of (*Bifidobacterium longum* (numbers 27 to 30 in Table 1).

**[Table 1]**

| Number | Strain name | Number | Strain name |
|---|---|---|---|
| 1 | *Lactobacillus plantarum* P1 | 16 | *Lactococcus lactis* P31 |
| 2 | *Lactobacillus plantarum* P3 | 17 | *Lactococcus lactis* P32 |
| 3 | *Lactobacillus plantarum* P4 | 18 | *Lactococcus lactis* P33 |
| 4 | *Lactobacillus plantarum* P5 | 19 | *Lactococcus lactis* P34 |
| 5 | *Lactobacillus plantarum* P6 | 20 | *Lactococcus lactis* P35 |
| 6 | *Lactobacillus sakei* P11 | 21 | *Bifidobacterium adolescentis* P42 |
| 7 | *Lactobacillus sakei* P12 | 22 | *Bifidobacterium adolescentis* P43 |
| 8 | *Lactobacillus sakei* P13 | 23 | *Bifidobacterium adolescentis* P44 |
| 9 | *Lactobacillus sakei* P15 | 24 | *Bifidobacterium bifidum* P45 |
| 10 | *Lactobacillus sakei* P17 | 25 | *Bifidobacterium bifidum* P46 |
| 11 | *Lactobacillus reuteri* P21 | 26 | *Bifidobacterium bifidum* P48 |
| 12 | *Lactobacillus reuteri* P22 | 27 | *Bifidobacterium longum* P51 |
| 13 | *Lactobacillus rhamnosus* P25 | 28 | *Bifidobacterium longum* P52 |
| 14 | *Lactobacillus rhamnosus* P28 | 29 | *Bifidobacterium longum* P54 |
| 15 | *Lactobacillus rhamnosus* P29 | 30 | *Bifidobacterium longum* P55 |

### 1-3. Physiological characteristics of Lactococcus lactis P32 which is novel lactic acid bacterium

Among the strains listed in Table 1 above, *Lactococcus lactis* P32 is a Gram-positive micrococcus, and hemolysis is not observed in the blood culture medium. The 16S rDNA of *Lactococcus lactis* P32 turned out to have the base sequence set forth in SEQ ID NO. 1, and as a result of comparing the base sequence of the 16S rDNA of *Lactococcus lactis* P32 with the base sequence of 16S rDNA by the BLAST search, it was confirmed that a *Lactococcus lactis* strain having the same base sequence of the 16S rDNA was not detected, and the base sequence of the 16S rDNA of *Lactococcus lactis* P32 showed 99% of homology with the base sequence of 16S rDNA of the publicly known *Lactococcus lactis* strain.

The utilization of carbon sources among the physiological characteristics of *Lactococcus lactis* P32 was analyzed with a sugar fermentation test using an API 50 CHL kit (BioMerieux's, USA). The results are shown in Table 2, where in Table 2, "+" indicates a case where the utilization of carbon sources is positive, and "-" indicates a case where the utilization of carbon sources is negative.

**[Table 2]**

| Carbon source | *Lactococcus lactis* P32 | Carbon source | *Lactococcus lactis* P32 |
|---|---|---|---|
| Control | - | Esculin | + |
| Glycerol | - | Salicin | + |
| Erythritol | - | Cellobiose | + |
| D-arabinose | - | Maltose | + |
| L-arabinose | - | Lactose | + |
| D-Ribose | + | Melibiose | - |
| D-xylose | + | Sucrose | + |
| L-xylose | - | Trehalose | + |
| D-adonitol | - | Inulin | - |
| Methyl-β-D-xylopyranoid | - | Melezitose | - |
| D-galactose | + | Raffinose | - |
| D-glucose | + | Starch | + |
| D-fructose | + | Glycogen | - |
| D-mannose | + | Xylitol | - |
| L-sorbose | - | Gentiobiose | + |
| Rhamnosus | - | D-turanose | - |
| Dulcitol | - | D-lyxose | - |
| Inositol | - | D-tagatose | - |
| Mannitol | + | D-fucose | - |
| Sorbitol | - | L-fucose | - |
| α-methyl-D-mannoside | - | D-arabitol | - |
| α-methyl-D-glucoside | - | L-arabitol | - |
| N-acetyl-glucosamine | + | Gluconate | - |
| Amygdalin | + | 2-keto-gluconate | - |
| Arbutin | + | 5-keto-gluconate | - |

The new strain, *Lactococcus lactis* P32, was subjected to patent deposition at the Korean Culture Center of Microorganisms, which is a depositary institution (address: Eurem Building, 45, Hongjenae 2ga-gil, Seodaemun-gu, Seoul, Republic of Korea), and an entrustment number of KCCM13222P was assigned.

### 1-4. Physiological characteristics of novel lactic acid bacterium, Bifidobacterium bifidum P45

*Bifidobacterium bifidum* P45 is a Gram-positive bacillus, and hemolysis is not observed in the blood culture medium. The 16S rDNA of *Bifidobacterium bifidum* P45 turned out to have the base sequence set forth in SEQ ID NO. 2, and as a result of comparing the base sequence of the 16S rDNA of *Bifidobacterium bifidum* P45 with the base sequence of 16S rDNA by the BLAST search, it was confirmed that a *Bifidobacterium bifidum strain* having the same base sequence of the 16S rDNA was not detected, and the base sequence of the 16S rDNA of *Bifidobacterium bifidum* P45 showed 99% of homology with the base sequence of 16S rDNA of the publicly known *Bifidobacterium bifidum* strain.

The utilization of carbon sources among the physiological characteristics of *Bifidobacterium bifidum* P45 was analyzed with a sugar fermentation test using an API 20 CHL kit (BioMerieux's, USA). The results and other physiological characteristics are shown in Table 3, where in Table 3, "+" indicates a case where the reaction is positive, and "-" indicates a case where the reaction is negative.

**[Table 3]**

| Carbon source | Reaction/enzyme | *Bifidobacterium bifidum* P45 |
|---|---|---|
| L-tryptophan | indole formation | - |
| Urea | urease | - |
| D-glucose | acidification (glucose) | + |
| D-mannitol | acidification (mannitol) | - |
| D-lactose | acidification (lactose) | + |
| D-saccharose | acidification (saccharose) | + |
| D-maltose | acidification (maltose) | + |
| Salicin | acidification (salicin) | - |
| D-xylose | acidification (xylose) | - |
| L-arabinose | acidification (arabinose) | + |
| Gelatin | hydrolysis (protease) | + |
| Eschulin sulfuric acid | hydrolysis (β-glucosidase) | + |
| Glycerol | acidification (glycerol) | - |
| D-cellobiose | acidification (cellobiose) | + |
| D-mannose | acidification (mannose) | + |
| D-melezitose | acidification (melezitose) | - |
| D-raffinose | acidification (raffinose) | + |
| D-sorbitol | acidification (sorbitol) | - |
| L-rhamnose | acidification (rhamnose) | - |
| D-trehalose | acidification (trehalose) | - |
| | Catalase | - |
| | Spores | - |
| | Gram reaction | + |
| | Morphology | - |

The new strain, *Bifidobacterium bifidum* P45, was subjected to patent deposition at the Korean Culture Center of Microorganisms, which is a depositary institution (address: Eurem Building, 45, Hongjenae 2ga-gil, Seodaemun-gu, Seoul, Republic of Korea), and an entrustment number of KCCM13223P was assigned.

### Experimental Example 1. Checking of antibiotic susceptibility of Lactococcus lactis P32 strain and Bifidobacterium bifidum P45 strain

The MIC (minimum inhibition concentration) for antibiotics was determined by transplanting 2 × 10⁵ CFU/ml of *Lactococcus lactis* P32 and *Bifidobacterium bifidum* P45 in 2 ml of an antibiotic-containing brain heart infusion (BHI) culture medium (BD Biosciences Korea, Seoul, South Korea) and carrying out culture anaerobically at 37°C for 24 hours, and the results are shown in Table 4.

**[Table 4]**

| | Minimum growth inhibition concentration (MIC, µg/ml) | | | | | | | | |
|---|---|---|---|---|---|---|---|---|---|
| | AM | VM | GM | KM | SM | EM | ClM | TC | CM |
| *Lactococcus lactis* P32 | 0.5 | 1 | 4 | 16 | 32 | 0.5 | 0.5 | 0.5 | 8 |
| *Bifidobacterium bifidum* P45 | 0.5 | 1 | 16 | 32 | 16 | 0.5 | 0.5 | 0.5 | 1 |

| | | | | | | | | | |
|---|---|---|---|---|---|---|---|---|---|
| * AM, ampicillin; VM, vancomycin; GM, gentamicin; KM, kanamycin; SM, streptomycin; EM, erythromycin; ClM, clindamycin; TC, tetracycline; and CM, chloramphenicol | | | | | | | | | |

### Experimental Example 2. Checking of antimicrobial effect of lactic acid bacteria according to the present invention on causative bacterium of vaginitis

A BHIS culture medium which had been obtained by adding 1% yeast extract (BD), 0.1% maltose, 0.1% glucose, and 10% horse serum to a brain heart infusion (BHI) culture medium (BD Biosciences Korea, Seoul, South Korea) was prepared. The isolated lactic acid bacteria (1 × 10⁶ CFU/ml), and *Gardnerella vaginalis* or *Staphylococcus aureus* (1 × 10⁶ CFU/ml) were transplanted in the BHIS culture medium and cultured for 24 hours at 37°C in anaerobic conditions. Then, the bacteria were collected, the bacterial DNA was extracted with a Qiagen DNA purification kit (Qiagen, Germany), and the strains of *Gardnerella vaginalis* and *Staphylococcus aureus* were quantified with qPCR.

Specifically, qPCR was carried out in a Thermal cycler (Qiagen) by adding extracted DNA (100 ng), a Sybr premix (Takara Bio, Japan), and primers. The primers in Table 5 were used, a treatment for 30 seconds at 95°C was carried out, and then 40 cycles of qPCR were carried out for 5 seconds at 95°C and for 30 sec at 72°C. The measurement results of the measurement of the antimicrobial effect are shown in Table 5.

**[Table 5]**

| SEQ ID NO. | Bacteria | Direction | Base sequence (5'-3') |
|---|---|---|---|
| 3 | *Gardnerella vaginalis* | Forward (F) | TTACTGGTGTATCACTGTAAGG |
| 4 | | Reverse (R) | CCGTCACAGGCTGAACAGT |
| 5 | *Staphylococcus aureus* | Forward (F) | ACGGTCTTGCTGTCACTTATA |
| 6 | | Reverse (R) | TACACATATGTTCTTCCCTAATAA |

### Experimental Example 3. Checking of inflammation suppressing efficacy of lactic acid bacteria according to the present invention

Ten mice of C57BL/6 mice (male, 6 weeks old, 19 to 22 g) per group were used. 2 ml of sterilized 4% thioglycolate was administered into the abdominal cavity of the mice, and after 4 days, the mice were anesthetized to administer 8 ml of an RPMI 1640 culture medium into the abdominal cavity of the mice. After 5 to 10 minutes, the RPMI culture medium (including macrophages) in the abdominal cavity of the mice were removed again and centrifuged for 10 minutes at 1,000 rpm, and then washing was carried out again twice with the RPMI 1640 culture medium. Macrophages were plated on a 24-well plate so that the number thereof was 0.5 × 10⁶ per well, lactic acid bacteria and the heat-treated inactivated G. vaginalis (1 × 10⁵ CFU/ml), which is an inflammatory response inducing substance, were placed therein, and the culture was carried out in a CO₂/air incubator for 24 hours. The supernatant was obtained to measure the expression levels of TNF-α, IL-1β, and IL-10 cytokine with an enzyme-linked immunosorbent assay (ELISA) kit (eBioscience, San Diego, CA, USA).

Specifically, first, 0.05 ml of the supernatant was placed in a 96-well plate which had been subjected to coating with each of antibodies against TNF-α, IL-1β, and IL-10. The reaction was allowed to proceed at an ordinary temperature for 2 hours, washing was subsequently carried out with phosphate buffered saline-Tween (PBS-Tween), a color developer was added thereto to allow coloration to proceed for 20 minutes, and the absorption at 450 nm was measured, followed by calculation. In addition, the quantity of cells attached to the well plate was measured by the MTT method. The results of the inflammation suppressing efficacy test for the lactic acid bacteria are shown in Table 6.

### Experimental Example 4. Effect of expression of claudin-1 protein in Caco2 cells

Caco2 cells were distributed from the Korean Cell Line Bank and cultured in an RPMI 1640 culture medium for 48 hours, and then the caco2 cells were aliquoted into 2 × 10⁶ cells per well. Thereafter, isolated lactic acid bacteria (1 × 10⁴ CFU/ml) were placed in each well, and together with them, 20 ng/ml of *G. vaginalis* (1 × 10⁵ CFU/ml) were added thereto. Then, the culture was carried out for 24 hours in a CO₂/air incubator. Thereafter, the cells were collected, a RIPA buffer was added thereto to lyze the cells, and then centrifugal separation was carried out (10,000g, 10 min). The supernatant was separated, and the expression level of claudin-1 was measured using an enzyme-linked immunosorbent assay (ELISA) kit (eBioscience, San Diego, CA, USA). The method in Experimental Example 3 described above is referenced. The expression level of claudin-1 in each lactic acid bacterium is as shown in Table 6 below.

**[Table 6]**

| | Inhibition rate (%)¹⁾ | | | | Increase rate (%) |
|---|---|---|---|---|---|
| | *Gardnerella vaginalis* | *Staphylococcus aureus* | TNF-α/ IL-10 | IL-1β/ IL10 | Claudin-1 |
| *Lactobacillus plantarum* P1 | + | + | + | + | - |
| *Lactobacillus plantarum* P3 | - | - | ++ | ++ | - |
| *Lactobacillus plantarum* P4 | ++ | + | ++ | +++ | ++ |
| *Lactobacillus plantarum* P5 | - | - | + | + | + |
| *Lactobacillus plantarum* P6 | + | - | + | + | + |
| *Lactobacillus sakei* P11 | ++ | + | +++ | ++ | ++ |
| *Lactobacillus sakei* P12 | - | - | + | + | - |
| *Lactobacillus sakei* P13 | - | - | + | + | + |
| *Lactobacillus sakei* P15 | - | - | - | - | + |
| *Lactobacillus sakei* P17 | - | - | + | + | - |
| *Lactobacillus reuteri* P21 | + | - | + | + | - |
| *Lactobacillus reuteri* P22 | + | - | + | + | + |
| *Lactobacillus rhamnosus* P25 | + | - | + | - | + |
| *Lactobacillus rhamnosus* P28 | ++ | + | + | + | + |
| *Lactobacillus rhamnosus* P29 | ++ | + | ++ | ++ | ++ |
| *Lactococcus lactis* P31 | ++ | + | + | + | + |
| *Lactococcus lactis* P32 | +++++ | ++ | +++ | ++++ | +++ |
| *Lactococcus lactis* P33 | + | + | ++ | ++ | - |
| *Lactococcus lactis* P34 | + | + | + | + | + |
| *Lactococcus lactis* P35 | + | - | + | + | |
| *Bifidobacterium adolescentis* P42 | - | - | + | + | |
| *Bifidobacterium adolescentis* P43 | - | - | - | + | + |
| *Bifidobacterium adolescentis* P44 | - | - | - | - | + |
| *Bifidobacterium bifidum* P45 | ++ | ++ | +++ | +++ | ++++ |
| *Bifidobacterium bifidum* P46 | + | - | + | + | + |
| *Bifidobacterium bifidum* P48 | - | - | - | - | + |
| *Bifidobacterium longum* P51 | + | + | ++ | ++ | + |
| *Bifidobacterium longum* P52 | - | - | + | + | + |
| *Bifidobacterium longum* P54 | - | - | + | + | + |
| *Bifidobacterium longum* P55 | + | - | + | + | + |

| | | | | | |
|---|---|---|---|---|---|
| 1) Inhibition rate: -, <5% inhibition; +, 5% to 20% inhibition; ++, 21% to 40% inhibition; +++, 41% to 60% inhibition; ++++, 61% to 80% inhibition; +++++, 81% to 100% inhibition. Inhibition rate (%) = 100 × ([group treated with only *G. vaginalis* or *S. aureus* - group treated with lactic acid bacteria and *G. vaginalis* or *S. aureus]* / [group treated with only *G. vaginalis* or *S. aureus* - group treated with only vehicle]) 2) increase rate: -, <5% increase; +, 5% to 20% increase; ++, 21% to 40% increase; +++, 41% to 60% increase; ++++, 61% to 80% increase; +++++, 81% to 100% increase. Induction/Increase (%) = 100 × ([group treated with lactic acid bacteria and *G. vaginalis* - group treated with only G. *vaginalis*] / [group treated with only vehicle - group treated with only *G. vaginalis*]) | | | | | |

As shown in Table 6 above, *Lactococcus lactis* P32 and *Bifidobacterium bifidum* P45 among the isolated lactic acid bacteria have excellent antimicrobial efficacy against *Gardnerella vaginalis* or *Staphylococcus aureus.* In addition, in the mice that induced the inflammatory response, the numerical value of the inflammatory cytokine (TNF-α or IL-1β) was reduced as compared with the anti-inflammatory cytokine (IL-10). This suggests that *Lactococcus lactis* P32 and *Bifidobacterium bifidum* P45 are lactic acid bacteria that have excellent antimicrobial and anti-inflammatory effects.

### Experimental Example 5. Screening of lactic acid bacteria for ameliorating vaginitis

### 5-1. Preparation of vaginitis model mouse and administration of lactic acid bacteria

C57BL/6 mice (female, 18 to 21 g, 6 weeks old) were adapted in the laboratory for one week and then divided into seven groups, where one group consisted of five mice, and the experiment was carried out. The mice were subjected to subcutaneous injection with 0.125 mg of β-estradiol 17-benzoate (Sigma-Aldrich Corporation, MO, USA) which had been dissolved in olive oil and after 72 hours, the inside of the vagina was infected with *Gardnerella vaginalis* (1 × 10⁸ CFU/mouse/20 µL). 1 × 10⁹ CFU/mouse of lactic acid bacteria of *Lactobacillus plantarum* P4, *Lactobacillus sakei* P11, *Lactococcus lactis* P32, *Bifidobacterium bifidum* P45, or *Bifidobacterium longum* P51 was orally administered once daily for 14 days from the 8th day after the infection. Lactic acid bacteria were administered for 14 days, and after 24 hours, a behavioral experiment was carried out for 2 days. On the next day, the mice were sacrificed to separate blood, and the vagina was washed to obtain a vagina lavage fluid. Subsequently, the vagina tissue, the colon tissue, and the brain tissue were separated.

### 5-2. Checking of vaginitis occurrence and quantification of Gardnerella vaginalis strain

In the vagina and the womb infected with *Gardnerella vaginalis,* the degree of occurrence of inflammation accompanied by edema was checked externally. DNA was isolated from the lavage fluid obtained from the mice with a Qiagen DNA purification kit (Qiagen, Germany), and *Gardnerella vaginalis* was quantified with PCR. PCR was carried out in the same manner as in Experimental Example 2. The measurement results are shown in Table 7 below.

### 5-3. Analysis of cytokines in vagina, colon, and hippocampus

A RIPA lysis buffer was added to the vagina, the colon, and the hippocampus, which had been separated from the mice, homogenization was carried out, and centrifugal separation was carried out to obtain supernatant. Then, an ELISA kit was used to measure the amounts of TNF-α, IL-1β, IL-6, IL-10, and BDNF. The amount of protein was measured using the Bradford assay. In addition, the amounts of corticosterone and IL-6 in the blood were also measured using an ELISA kit. For the above-described measurement method, the method in Experimental Example 3 is referenced. The measurement results are shown in Table 7 below.

### 5-4. Behavioral experiment

### 5-4-1. Elevated plus maze (EPM) test

An elevated plus maze experiment device is a device in which two open arms (30 × 7 cm) and two enclosed arms (30 × 7 cm) having a wall having a height of 20 cm extend respectively by 7 cm (7 × 7 cm) from the central platform. It is made of black flexible glass and has a wall that is higher than the floor by 50 cm. In a room at a brightness of 20 lux, equipped with a video camera on the top of the room, the movement of the mouse in the elevated plus maze was recorded.

The mouse was placed towards the open arms so that the head thereof was at the center of the elevated plus maze (EPM), and then the times and frequencies spent in the open arms and the enclosed arms were measured for 5 minutes. The arm entry was defined such that all four feet were in the arm. The time spent in the open arms during the entire experimental time was (Time spent in open arms)/(time spent in open arms + time spent in enclosed arms) × 100) and was calculated for each mouse. After the end of each behavioral experiment, the remaining smell was removed with 70% ethanol. The measurement results are shown in Table 7 below.

### 5-4-2. Tail suspension test (TST)

1 cm of the mouse tail end was hung at the center of a container having a diameter of 35 cm and a height of 50 cm with a fixing device. The immobility time of the mouse was measured for a total of 6 minutes. Immobility refers to a state of without movement but with only minimal action. The measurement results are shown in Table 7 below.

### 5-4-3. Forced swimming test (FST)

A cylindrical water bath having a height of 40 cm and a diameter of 20 cm was filled with water at a temperature of 25 ± 1°C to a height of 30 cm. The mice were put in the water bath one by one. The first 2 minutes of the total 6 minutes corresponded to the adaptation time, and thus time the measurement was not carried out in this period. The immobility time of the experimental animal during the last 4 minutes was measured. The immobility means a state in which a mouse stands upright and floats without movement while making only minimal movement to expose only the head out of the water. The measurement results are shown in Table 7 below.

**[Table 7]**

| | Inhibition rate (%)¹⁾ | | | | | | | | | | | | |
|---|---|---|---|---|---|---|---|---|---|---|---|---|---|
| | Vagina | | | Blood | | Colon | | Hippocampus | | | Behavior | | |
| | GV | TNF-a | IL -6 | IL-6 | Co rt ic os te ro ne | TNF-a | IL -6 | BNDF | TNF-a | IL -6 | EPM | TST | FST |
| *Lact obac illu s plan taru m* P4 | + | ++ | ++ | + | + | ++ | + | + | + | + | ++ | ++ | + |
| *Lact obac illu s sake i* P11 | + | ++ | + | + | + | ++ | + | - | - | + | ++ | ++ | ++ |
| *Lact ococ cus lact is* P32 | ++ + | ++++ | ++ + | +++ | ++ + | ++++ | ++ + | +++ | +++ | ++ + | +++ | ++++ | ++++ |
| *Bifi doba cter ium bifidum* P45 | ++ | ++ | ++ | ++ | ++ | +++ | ++ | +++ | +++ | ++ + | +++ | ++++ | ++++ |
| *Bifi doba cter ium long um* P51 | + | + | + | + | + | + | ++ | ++ | + | ++ | + | ++ | + |

| | | | | | | | | | | | | | |
|---|---|---|---|---|---|---|---|---|---|---|---|---|---|
| 1) Inhibition rate: -, <5% inhibition; +, 5% to 20% inhibition; ++, 21% to 40% inhibition; +++, 41% to 60% inhibition; ++++, 61% to 80% inhibition; +++++, 81% to 100% inhibition. Inhibition rate (%) = 100 × ([group treated with only *G. vaginalis* or *S. aureus* - group treated with lactic acid bacteria and *G. vaginalis* or *S. aureus*] / [group treated with only *G. vaginalis* or *S. aureus* - group treated with only vehicle]) | | | | | | | | | | | | | |

As shown in Table 7 above, *Lactococcus lactis* P32 and *Bifidobacterium bifidum* P45 among the isolated lactic acid bacteria had an excellent growth inhibitory effect on *Gardnerella vaginalis* in the vagina and significantly reduced numerical values of inflammatory cytokines in the vagina, the blood, the colon, and the hippocampus.

In addition, the time and frequency spent in the open arms in the elevated plus maze in the lactic acid bacteria administration group were increased, and the time in immobility was markedly reduced in the forced swimming test and the tail suspension test.

This suggests that *Lactococcus lactis* P32 and *Bifidobacterium bifidum* P45 are strains that not only have an excellent effect on the prevention and treatment of an inflammatory disease but also are effective in the amelioration of depressive disorder, anxiety disorder, and the like.

### Experimental Example 6. Confirmation of the amelioration effects of Lactococcus lactis P32, Bifidobacterium bifidum P45, and their mixed strains on vaginitis in a mouse model induced with vaginitis

### 6-1. Preparation of vaginitis-induced mouse and administration of lactic acid bacteria

C57BL/6 mice (female, 18 to 21 g, 6 weeks old) were divided into five groups, where one group consisted of five mice, and the mice were adapted to the laboratory for one week. The mice were subjected to subcutaneous injection with 0.125 mg of β-estradiol 17-benzoate (Sigma-Aldrich Corporation, MO, USA) which had been dissolved in olive oil and after 72 hours, the inside of the vagina was infected with *Gardnerella vaginalis* (1 × 10⁸ CFU/mouse/20 µL) once daily for 7 days. The negative control group (NC) was treated with only the physiological saline that had been used for the suspension of *Gardnerella vaginalis,* and the positive control group (GV) was not administered with lactic acid bacteria after being infected with *Gardnerella vaginalis.*

The experimental group was orally administered once daily for 14 days from the 8th day after the infection, with 1 × 10⁹ CFU/mouse of *Lactococcus lactis* P32 and *Bifidobacterium bifidum* P45, which are the isolated lactic acid bacteria, and mixed strains (*Lactococcus lactis P32:Bifidobacterium bifidum* P45 = 4:1).

Lactic acid bacteria were administered for 14 days, and after 24 hours, a behavioral experiment was carried out for 2 days. On the next day, the mice were sacrificed to separate blood, and the vagina was washed to obtain a vagina lavage fluid. Subsequently, the blood, the vagina, the colon, and the hippocampus were separated.

### 6-2. Confirmation of amelioration effect on vaginitis and quantification of Gardnerella vaginalis strain

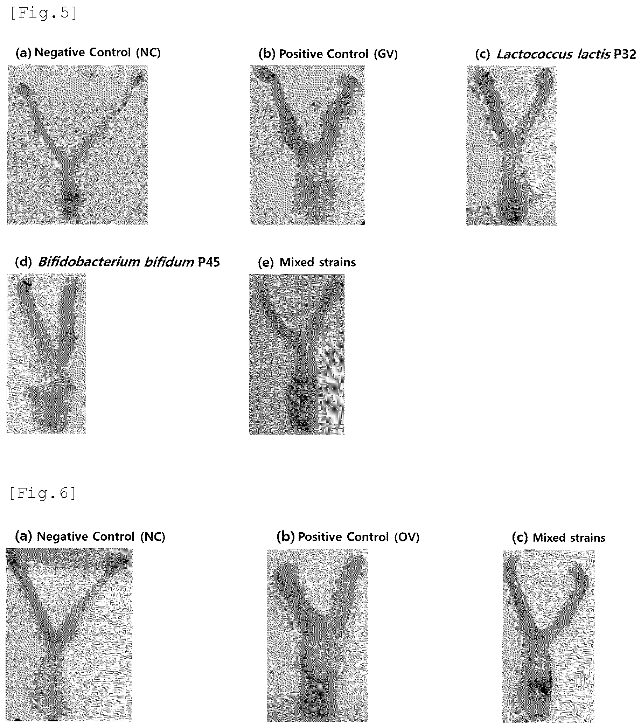

The occurrence of inflammation and the amelioration effect due to the administration of lactic acid bacteria were confirmed in the vagina separated from the mouse. In addition, the *Gardnerella vaginalis* strain was quantified in the vagina. The quantification was carried out in the same manner as in Experimental Example 5, and the measurement results are shown in Fig. 5 and Table 8 below.

As shown in Fig. 5 and Table 8 below, inflammation occurred in the positive control group (GV) infected with *Gardnerella vaginalis,* and inflammation was ameliorated in a case where the lactic acid bacteria according to the present invention were administered. In addition, in a case where the lactic acid bacteria according to the present invention were administered, the *Gardnerella vaginalis* strain was reduced in the vagina. This suggests that *Lactococcus lactis* P32, *Bifidobacterium bifidum* P45, and mixed strains thereof are strains effective for prevention or amelioration of inflammation, and prevention or treatment of *Gardnerella vaginalis* infection.

### 6-3. Analysis of cytokines in vagina, colon, blood, and hippocampus

A RIPA lysis buffer was added to the vagina, the colon, and the hippocampus, which had been separated from the mice, homogenization was carried out, and centrifugal separation was carried out to obtain supernatant, which was used for the analysis of cytokines and the like. The expression levels of TNF-α, IL-1β, IL-6, and IL-10 in the vagina supernatant, the expression levels of TNF-α, IL-1β, IL-6, and IL-10 in the colon supernatant, and the expression levels of TNF-α, IL-1β, IL-6, IL-10, and BDNF in the hippocampus supernatant were measured using an ELISA kit. The amounts of corticosterone and IL-6 in the blood were also measured using an ELISA kit. For the above-described measurement method, the method in Experimental Example 3 is referenced.

In addition, the expression level of claudin-1 in the vagina supernatant and the colon supernatant, and the expression level of claudin-5 in the hippocampus supernatant were measured by immunoblotting, and the protein amounts thereof were measured by the Bradford assay.

Specifically, 50 µg of the supernatant was taken, and electrophoresis was carried out on a polyacrylamide gel of SDS 10% (w/v) for 1 hour and 30 minutes. The sample subjected to electrophoresis was transferred to nitrocellulose paper for 1 hour and 10 minutes under conditions of 100 V and 400 mA. After blocking the nitrocellulose paper to which the sample had been transferred, with 5% skim milk for 30 minutes, it was washed three times for 5 minutes with phosphate buffered saline-Twin, and an anti-claudin-1 antibody or an anti-claudin-5 antibody (Santa Cruz Biotechnology, USA) was added thereto at a ratio of 1:100 and was allowed to react overnight. Subsequently, washing was carried out three times for 10 minutes, and a secondary antibody (Santa Cruz Biotechnology, USA) was added thereto at a ratio of 1:1,000 and allowed to react for 1 hour and 20 minutes. Subsequently, washing was carried out three times for 15 minutes, fluorescent coloration and then development were carried out, and the intensity of the colored band was measured. The measurement results are shown in Table 8 below.

### 6-4. Behavioral experiment

Similar to Experimental Example 5 described above, the elevated plus maze (EPM) test, the tail suspension test (TST), and the forced swimming test (FST) were carried out. The measurement results are shown in Table 8 below.

**[Table 8]**

| Tissue | Biomarker | Negative control group (NC) | Positive control group (GV) | *Lactococ cus lactis* P32 | *Bifidoba cterium bifidum* P45 | Mixed strains |
|---|---|---|---|---|---|---|
| Vagina | GV | 1 ± 0.4 | 23.8 ± 10.2 | 6.5 ± 4.3 | 14.3 ± 7.5 | 5.6 ± 3.9 |
| | TNF-α | 2.8 ± 0.5 | 4.9 ± 0.4 | 3.3 ± 0.5 | 3.4 ± 0.4 | 3.0 ± 0.3 |
| | IL-1β | 9.9 ± 1.2 | 18.4 ± 2.4 | 11.2 ± 1.2 | 11.2 ± 1.5 | 10.9 ± 1.1 |
| | IL-6 | 1.4 ± 0.1 | 1.9 ± 0.2 | 1.5 ± 0.2 | 1.6 ± 0.1 | 1.4 ± 0.2 |
| | IL-10 | 0.33 ± 0.08 | 0.11 ± 0.05 | 0.25 ± 0.07 | 0.24 ± 0.08 | 0.27 ± 0.09 |
| | Claudin-1¹⁾ | 1 ± 0.11 | 0.58 ± 0.15 | 0.84 ± 0.12 | 0.80 ± 0.14 | 0.91 ± 0.12 |
| Colon | TNF-α | 3.5 ± 0.3 | 5.8 ± 0.7 | 4.2 ± 0.4 | 4.5 ± 0.3 | 4.0 ± 0.6 |
| | IL-1β | 12.4 ± 1.8 | 20.1 ± 1.2 | 14.5 ± 1.9 | 14.7 ± 1.2 | 14.1 ± 1.3 |
| | IL-6 | 2.2 ± 0.4 | 3.5 ± 0.5 | 2.4 ± 0.4 | 2.6 ± 0.7 | 2.3 ± 0.8 |
| | IL-10 | 0.24 ± 0.03 | 0.16 ± 0.05 | 0.22 ± 0.05 | 0.20 ± 0.03 | 0.23 ± 0.04 |
| | Claudin-1¹⁾ | 1 ± 0.09 | 0.72 ± 0.12 | 0.89 ± 0.16 | 0.90 ± 0.10 | 0.95 ± 0.09 |
| Blood | IL-6 | 4.2 ± 0.5 | 8.3 ± 0.7 | 5.3 ± 0.7 | 5.5 ± 0.3 | 5.1 ± 0.5 |
| | Corticost erone | 0.6 ± 0.2 | 1.4 ± 0.2 | 0.7 ± 0.3 | 0.8 ± 0.1 | 0.7 ± 0.2 |
| Hippocam pus | TNF-α | 3.9 ± 0.1 | 6.9 ± 0.3 | 4.3 ± 0.4 | 4.7 ± 0.5 | 4.3 ± 0.2 |
| | IL-1β | 12.3 ± 1.1 | 20.5 ± 0.9 | 14.6 ± 1.4 | 15.2 ± 1.1 | 14.3 ± 1.5 |
| | IL-6 | 1.3 ± 0.2 | 2.7 ± 0.3 | 1.5 ± 0.2 | 1.6 ± 0.3 | 1.5 ± 0.3 |
| | IL-10 | 0.4 ± 0.05 | 0.2 ± 0.04 | 0.3 ± 0.02 | 0.3 ± 0.05 | 0.3 ± 0.02 |
| | BDNF | 2.4 ± 0.2 | 1.5 ± 0.1 | 2.2 ± 0.3 | 2.1 ± 0.1 | 2.2 ± 0.2 |
| | Claudin-5¹⁾ | 1 ± 0.1 | 0.78 ± 0.11 | 0.91 ± 0.12 | 0.92 ± 0.14 | 0.94 ± 0.09 |
| Behavior | EPM | 21.3 ± 2.1 | 12.3 ± 1.2 | 18.2 ± 1.5 | 18.4 ± 2.1 | 20.2 ± 1.8 |
| | TST | 25.8 ± 2.1 | 43.7 ± 3.8 | 28.5 ± 2.4 | 29.2 ± 2.2 | 27.4 ± 1.9 |
| | FST | 27.7 ± 1.9 | 42.5 ± 3.1 | 29.6 ± 2.2 | 30.3 ± 2.6 | 29.1 ± 3.4 |

| | | | | | | |
|---|---|---|---|---|---|---|
| 1) The rise rate was relatively calculated by setting the negative control group (NC) group treated with only the vehicle as 1. | | | | | | |

As shown in Table 8 above, *Lactococcus lactis* P32, *Bifidobacterium bifidum* P45, or mixed strains thereof reduced the numerical values of inflammatory cytokines in the vagina, the colon, the blood, and the hippocampus, and increased the numerical values of anti-inflammatory cytokines.

In addition, in a case where the above-described lactic acid bacteria and the mixed strains thereof were administered, the time and frequency spent in the open arms in the elevated plus maze were increased, and the time in immobility was markedly reduced in the forced swimming test and the tail suspension test, and the numerical values of BDNF and Claudin-5 were increased in the hippocampus.

This suggests that *Lactococcus lactis* P32, *Bifidobacterium bifidum* P45, and mixed strains thereof are strains that not only have an excellent effect on the prevention or treatment of vaginitis but also are effective in the amelioration of depressive disorder or anxiety disorder due to an inflammatory disease.

### Experimental Example 7. Confirmation of amelioration effect of mixed strains of Lactococcus lactis P32 and Bifidobacterium bifidum P45 on osteoporosis in osteoporosis-induce mouse

### 7-1. Preparation of osteoporosis mouse and administration of lactic acid bacteria

C57BL/6 mice (female, 18 to 21 g, 6 weeks old) were divided into three groups, where one group consisted of seven mice, and the mice were adapted to the laboratory for one week. After anesthesia with isoflurane, ovariectomy was carried out to induce osteoporosis.

The negative control group (NC) was not subjected to ovariectomy, and the positive control group (OV) was not administered with lactic acid bacteria after being subjected to ovariectomy.

The experimental group was orally administered, once daily for four weeks (28 days), with 1 × 10⁹ CFU/mouse of mixed strains of *Lactococcus lactis* P32 and *Bifidobacterium bifidum* P45, which are the isolated lactic acid bacteria, and mixed strains (*Lactococcus lactis* P32:*Bifidobacterium bifidum* P45 = 4:1). The mice were administered with the last lactic acid bacteria. After 24 hours, a behavioral experiment was carried out for 2 days, and on the next day, the mice were sacrificed to separate blood. Subsequently, the vagina, the brain, the colon, and the femur were separated.

### 7-2. Confirmation of occurrence of vaginitis and amelioration effect on vaginitis

The occurrence of inflammation and the amelioration effect due to the administration of lactic acid bacteria were confirmed in the vagina separated from the mouse. The checking was carried out in the same manner as in Experimental Example 5, and the results are shown in Fig. 6 below.

As shown in Fig. 6 below, inflammation occurred in mice that had been subjected to ovariectomy, and inflammation was ameliorated in a case where the lactic acid bacteria according to the present invention was administered. This suggests that mixed strains of *Lactococcus lactis* P32 and *Bifidobacterium bifidum* P45 are strains that are effective in the prevention or amelioration of inflammation.

### 7-3. Analysis of cytokines in vagina, colon, blood, and hippocampus, and measurement of bone weight

A RIPA lysis buffer was added to the vagina, the colon, and the hippocampus, which had been separated from the mice, homogenization was carried out, and centrifugal separation was carried out to obtain supernatant, which was used for the analysis of cytokines and the like. The expression levels of TNF-α and IL-6 in the vagina supernatant, and the expression levels of TNF-α, IL-1β, and BDNF in the hippocampus supernatant were measured with an ELISA kit. For the above-described measurement method, the method in Experimental Example 3 is referenced. Corticosterone and IL-6 in the blood were measured with an ELISA kit and osteocalcin was measured with an ELISA kit from R&D system (Minneapolis, MN, USA). Calcium (Ca) was measured with ASAN Ca-Lq Reagents (ASAN PHARM. CO., LTD.). In addition, the bone weight of the femur was also measured. The measurement results are shown in Table 9 below.

### 7-4. Behavioral experiment

Similar to Experimental Example 5, the elevated plus maze (EPM) test, the tail suspension test (TST), and the forced swimming test (FST) were carried out. The measurement results are shown in Table 9 below.

**[Table 9]**

| Tissue | Biomarker | Negative control group (NC) | Positive control group (OV) | Mixed strains |
|---|---|---|---|---|
| Vagina | TNF-α (pg/mg) | 2.9 ± 0.4 | 4.5 ± 0.5 | 3.3 ± 0.3 |
| | IL-6 | 1.2 ± 0.1 | 1.6 ± 0.2 | 1.3 ± 0.1 |
| Blood | IL-6 (pg/mL) | 3.3 ± 0.2 | 5.3 ± 0.4 | 4.1 ± 0.3 |
| | Corticosterone (ng/mL) | 0.5 ± 0.1 | 0.9 ± 0.2 | 0.6 ± 0.1 |
| | Calcium (mmol/L) | 0.29 ± 0.02 | 0.22 ± 0.03 | 0.25 ± 0.02 |
| | Osteocalcin (pg/mL) | 0.42 ± 0.04 | 0.25 ± 0.04 | 0.33 ± 0.01 |
| Hippocampus | TNF-α (pg/mg) | 3.1 ± 0.4 | 5.5 ± 0.2 | 3.9 ± 0.3 |
| | IL-6 | 1.6 ± 0.3 | 2.4 ± 0.3 | 1.9 ± 0.3 |
| | BDNF | 2.1 ± 0.2 | 1.4 ± 0.2 | 1.9 ± 0.3 |
| Behavior | EPM | 19.9 ± 2.1 | 11.3 ± 1.5 | 17.2 ± 1.3 |
| | TST | 22.4 ± 2.2 | 31.4 ± 2.3 | 25.6 ± 1.2 |
| | FST | 25.3 ± 2.5 | 34.6 ± 1.9 | 28.4 ± 2.1 |
| Bone | Femur (g) | 0.17 ± 0.02 | 0.14 ± 0.01 | 0.16 ± 0.02 |

As shown in Table 9 above, in a case where the mixed strains of *Lactococcus lactis* P32 and *Bifidobacterium bifidum* P45 were administered, the numerical values of the calcium and osteocalcin in the blood increased as compared with those in the positive controlled group that had not been administered with lactic acid bacteria, and the bone weight was increased.

In addition, in a case where the mixed strains of the lactic acid bacteria were administered, the time and frequency spent in the open arms in the elevated plus maze was increased. In the forced swimming test and the tail suspension test, the time in immobility was markedly reduced, and the numerical value of BDNF was increased in the hippocampus.

This suggests that *Lactococcus lactis* P32, *Bifidobacterium bifidum* P45, and mixed strains thereof are strains that have an excellent effect on the prevention or treatment of osteoporosis and are effective in the amelioration of depressive disorder and anxiety disorder due to the female hormone imbalance.

### Experimental Example 8. Confirmation of the amelioration effects of Lactococcus lactis P32, Bifidobacterium bifidum P45, and their mixed strains on vaginitis and osteoporosis in a mouse model induced with both conditions.

### 8-1. Preparation of mouse with vaginitis and osteoporosis and administration of lactic acid bacteria

Seven mice of C57BL/6 mice (female, 21 to 23g, 6 weeks old) were adapted to the laboratory for one week. The mice were anesthetized with isoflurane, and then ovariectomy was carried out to induce osteoporosis. On the next day, the mice were subjected to subcutaneous injection with 0.125 mg of β-estradiol 17-benzoate (Sigma-Aldrich Corporation, MO, USA) which had been dissolved in olive oil and after 72 hours, the inside of the vagina was infected with Gardnerella vaginalis (1 × 10⁸ CFU/mouse/20 µL) once daily for 7 days.

The negative control group (NC) was treated with only the physiological saline that had been used for the suspension of Gardnerella vaginalis, and the positive control group (GV/OV) was not administered with lactic acid bacteria after being infected with Gardnerella vaginalis.

The experimental group was orally administered, once daily for 4 weeks (28 days) from the 8th day after the infection, with 1 × 10⁹ CFU/mouse of *Lactococcus lactis* P32 and *Bifidobacterium bifidum* P45, which are the isolated lactic acid bacteria, and mixed strains (*Lactococcus lactis* P32:*Bifidobacterium bifidum* P45 = 4:1). The last lactic acid bacteria were administered, and after 24 hours, a behavioral experiment was carried out for 2 days. On the next day, the mice were sacrificed to separate blood, and the vagina was washed to obtain a vagina lavage fluid. Subsequently, the blood, the vagina, the colon, and the brain were separated.

### 8-2. Confirmation of amelioration effect on vaginitis and quantification of Gardnerella vaginalis strain

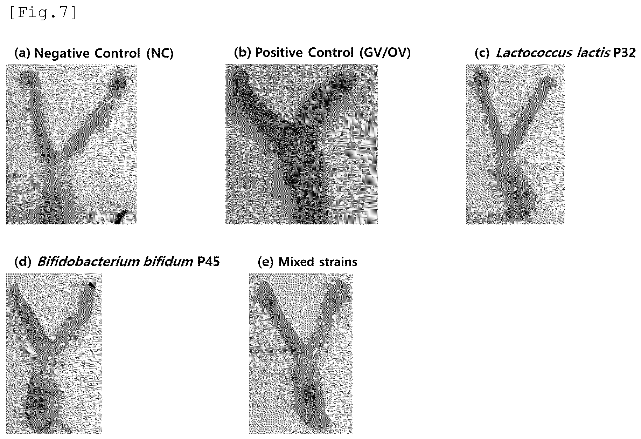

The occurrence of inflammation and the amelioration effect due to the administration of lactic acid bacteria were confirmed in the vagina separated from the mouse. In addition, the *Gardnerella vaginalis* strain was quantified in the vagina. The quantification was carried out in the same manner as in Experimental Example 5, and the measurement results are shown in Fig. 7 and Table 10 below.

As shown in Fig. 7 and Table 10, inflammation occurred in the positive control group (GV/OV) that had been infected with *Gardnerella vaginalis* and subjected to ovariectomy, and inflammation was ameliorated in a case where the lactic acid bacteria according to the present invention were administered. In addition, in a case where the lactic acid bacteria according to the present invention were administered, the *Gardnerella vaginalis* strain was reduced in the vagina. This suggests that *Lactococcus lactis* P32, *Bifidobacterium bifidum* P45, and mixed strains thereof are strains effective for prevention or amelioration of inflammation, and prevention or treatment of Gardnerella vaginalis infection.

### 8-3. Analysis of cytokines in vagina, colon, blood, and hippocampus, and measurement of bone weight

A RIPA lysis buffer was added to the vagina, the colon, and the hippocampus, which had been separated from the mice, homogenization was carried out, and centrifugal separation was carried out to obtain supernatant, which was used for the analysis of cytokines and the like. The expression levels of TNF-α, IL-1β, IL-6, and IL-10 in the vagina supernatant, the expression levels of TNF-α, IL-1β, IL-6, and IL-10 in the colon supernatant, and the expression levels of TNF-α, IL-1β, IL-6, IL-10, and BDNF in the hippocampus supernatant were measured with an ELISA kit. For the above-described measurement method, the method in Experimental Example 3 is referenced.

In addition, the expression level of claudin-1 in the vagina supernatant and the colon supernatant, and the expression level of claudin-5 in the hippocampus supernatant were measured by immunoblotting, and the protein amounts thereof were measured by the Bradford assay. For the above-described measurement method, the method in Experimental Example 6 is referenced.

Corticosterone and IL-6 in the blood were measured with an ELISA kit and osteocalcin was measured with an ELISA kit from R&D system (Minneapolis, MN, USA). Calcium (Ca) and phosphorous (P) were measured with ASAN Ca-Lq Reagents (ASAN PHARM. CO., LTD.) and ASAN Pi-Lq Reagents (ASAN PHARM. CO., LTD.), respectively. The protein amount was measured with a Bradford assay method. In addition, the bone weight of the femur was also measured. The measurement results are shown in Table 10.

### 8-4. Behavioral experiment

Similar to Experimental Example 5, the elevated plus maze (EPM) test, the tail suspension test (TST), and the forced swimming test (FST) were carried out. The measurement results are shown in Table 10 below.

**[Table 10]**

| Tissue | Biomarker | Negative control group (NC) | Positive control group (GV/OV) | *Lactococc us lactis* P32 | *Bifidobac terium bifidum* P45 | Mixed strains |
|---|---|---|---|---|---|---|
| Vagina | GV¹⁾ | 1 ± 0.2 | 34.3 ± 5.9 | 7.3 ± 3.5 | 12.2 ± 5.1 | 7.1 ± 2.9 |
| | TNF-α (pg/mg) | 3.2 ± 0.6 | 5.5 ± 0.9 | 3.8 ± 0.5 | 4.1 ± 0.5 | 3.7 ± 0.8 |
| | IL-1β | 12.1 ± 1.8 | 21.6 ± 2.3 | 15.2 ± 1.6 | 16.4 ± 1.9 | 15.5 ± 1.3 |
| | IL-6 | 1.3 ± 0.2 | 1.9 ± 0.3 | 1.5 ± 0.1 | 1.5 ± 0.3 | 1.4 ± 0.2 |
| | IL-10 (pg/mg) | 0.21 ± 0.03 | 0.08 ± 0.02 | 0.13 ± 0.04 | 0.14 ± 0.04 | 0.13 ± 0.04 |
| | Claudin-1¹⁾ | 1 ± 0.20 | 0.47 ± 0.21 | 0.81 ± 0.11 | 0.85 ± 0.12 | 0.89 ± 0.12 |
| Colon | TNF-α (pg/mL) | 4.4 ± 0.5 | 6.3 ± 0.8 | 4.4 ± 0.5 | 4.4 ± 0.5 | 4.4 ± 0.5 |
| | IL-1β | 14.2 ± 1.6 | 23.8 ± 1.5 | 18.2 ± 1.5 | 19.2 ± 1.9 | 17.3 ± 1.2 |
| | IL-6 | 2.4 ± 0.5 | 3.9 ± 0.6 | 2.9 ± 0.6 | 2.8 ± 0.4 | 2.8 ± 0.9 |
| | IL-10 | 0.25 ± 0.02 | 0.18 ± 0.03 | 0.20 ± 0.02 | 0.21 ± 0.02 | 0.20 ± 0.03 |
| | Claudin-1¹⁾ | 1 ± 0.14 | 0.53 ± 0.11 | 0.84 ± 0.12 | 0.91 ± 0.10 | 0.92 ± 0.09 |
| Blood | IL-6 (pg/mL) | 5.6 ± 0.6 | 10.2 ± 1.0 | 6.5 ± 0.4 | 7.3 ± 0.7 | 6.7 ± 0.5 |
| | Corticost erone (ng/mL) | 0.8 ± 0.2 | 1.8 ± 0.3 | 1.2 ± 0.2 | 0.3 ± 0.2 | 1.1 ± 0.3 |
| | Calcium (mmol/L) | 0.30 ± 0.03 | 0.21 ± 0.02 | 0.25 ± 0.02 | 0.25 ± 0.03 | 0.26 ± 0.02 |
| | Phosphoru s (mmol/L) | 0.8 ± 0.05 | 0.6 ± 0.04 | 0.7 ± 0.02 | 0.7 ± 0.01 | 0.7 ± 0.03 |
| | Osteocalc in (pg/mL) | 0.41 ± 0.03 | 0.15 ± 0.06 | 0.26 ± 0.05 | 0.25 ± 0.02 | 0.31 ± 0.05 |
| Hippocamp us | TNF-α (pg/mg) | 3.4 ± 0.3 | 8.5 ± 0.4 | 4.7 ± 0.2 | 4.8 ± 0.3 | 4.5 ± 0.4 |
| | IL-1β | 16.7 ± 1.4 | 27.1 ± 1.7 | 19.6 ± 1.2 | 21.2 ± 1.9 | 19.2 ± 1.1 |
| | IL-6 | 1.8 ± 0.2 | 3.3 ± 0.2 | 2.3 ± 0.4 | 2.5 ± 0.3 | 2.3 ± 0.3 |
| | IL-10 | 0.5 ± 0.03 | 0.1 ± 0.03 | 0.3 ± 0.02 | 0.3 ± 0.04 | 0.4 ± 0.02 |
| | BDNF | 2.2 ± 0.2 | 0.9 ± 0.1 | 1.8 ± 0.3 | 1.7 ± 0.2 | 1.9 ± 0.4 |
| | Claudin-5¹⁾ | 1 ± 0.1 | 0.74 ± 0.1 | 0.93 ± 0.06 | 0.89 ± 0.07 | 0.92 ± 0.03 |
| Behavior | EPM | 22.5 ± 2.5 | 10.4 ± 1.5 | 21.1 ± 2.1 | 20.2 ± 1.8 | 21.5 ± 1.7 |
| | TST | 24.4 ± 2.1 | 48.3 ± 3.1 | 26.4 ± 1.4 | 27.8 ± 2.3 | 26.1 ± 1.6 |
| | FST | 28.6 ± 2.4 | 49.8 ± 2.9 | 31.2 ± 2.2 | 32.1 ± 2.7 | 21.8 ± 1.4 |
| Bone | Femur (g) | 0.18 ± 0.02 | 0.13 ± 0.01 | 0.15 ± 0.01 | 0.15 ± 0.02 | 0.16 ± 0.02 |

| | | | | | | |
|---|---|---|---|---|---|---|
| 1) The rise rate was relatively calculated by setting the negative control group (NC) group treated with only the vehicle as 1. | | | | | | |

As shown in Table 10 above, *Lactococcus lactis* P32, *Bifidobacterium bifidum* P45, and mixed strains thereof reduced the numerical values of inflammatory cytokines in the vagina, the colon, the blood, and the hippocampus, and increased the numerical values of anti-inflammatory cytokines. In addition, the numerical values of the calcium, phosphorus, and osteocalcin in the blood increased, and the bone weight increased.

On the other hand, in a case where the above-described lactic acid bacteria and the mixed strains were administered, the time and frequency spent in the open arms in the elevated plus maze were increased, and the time in immobility was markedly reduced in the forced swimming test and the tail suspension test, and the numerical values of BDNF and Claudin-5 were increased in the hippocampus.

This suggests that *Lactococcus lactis* P32, *Bifidobacterium bifidum* P45, and mixed strains thereof are strains that have an excellent effect on the prevention or treatment of vaginitis and osteoporosis and are effective in the amelioration of an inflammatory disease or depressive disorder and anxiety disorder due to the female hormone imbalance.

The explanatory description of the present invention described above has been made exemplary, and thus those skilled in the art to which the present invention belongs shall be able to understand that the present invention can be easily modified in other specific forms without changing the technical ideas or essential features. Therefore, it should be understood that the examples described above should be understood as exemplary and be not limited thereto. For example, each constituent element described as being in a single form may be implemented in a distributed form, and similarly, constituent components described as being distributed may be implemented in a combined form.

The scope of the present invention is represented by the claims described below, and the meaning and the scope of the claims and any change or modified form derived from the concept equivalent thereto shall be construed as being included within the scope of the present invention.

Depositary institution: Korean Culture Center of Microorganisms (International)
Deposit number: KCCM13222P
Deposit date: 20220803
Depositary institution: Korean Culture Center of Microorganisms (International)
Deposit number: KCCM13223P
Deposit date: 20220803

## Claims

1. *Lactococcus lactis* P32 KCCM13222P.

2. The *Lactococcus lactis* P32 according to claim 1, wherein the *Lactococcus lactis* P32 KCCM13222P comprises a base sequence of 16S rDNA, which is set forth in SEQ ID NO. 1.

3. *Bifidobacterium bifidum* P45 KCCM13223P.

4. The *Bifidobacterium bifidum* P45 KCCM13223P according to claim 3, wherein the *Bifidobacterium bifidum* P45 KCCM13223P comprises a base sequence of 16S rDNA, which is set forth in SEQ ID NO. 2.

5. An antimicrobial composition comprising:
*Lactococcus lactis* P32 KCCM13222P;
*Bifidobacterium bifidum* P45 KCCM13223P; or
a mixture thereof.

6. A pharmaceutical composition for prevention or treatment of infection with *Gardnerella vaginalis* or *Staphylococcus aureus,* the pharmaceutical composition comprising the composition according to claim 5.

7. A pharmaceutical composition for prevention or treatment of an inflammatory disease or osteoporosis, the pharmaceutical composition comprising:
*Lactococcus lactis* P32 KCCM13222P;
*Bifidobacterium bifidum* P45 KCCM13223P; or
a mixture thereof.

8. The pharmaceutical composition according to claim 7, wherein the inflammatory disease is vaginitis.

9. A pharmaceutical composition for prevention or treatment of depressive disorder or anxiety disorder, the pharmaceutical composition comprising:
*Lactococcus lactis* P32 KCCM13222P;
*Bifidobacterium bifidum* P45 KCCM13223P; or
a mixture thereof.

10. The pharmaceutical composition according to claim 9, wherein the depressive disorder or the anxiety disorder is caused by an inflammatory disease or a female hormone imbalance.

11. The pharmaceutical composition according to any one of claims 6 to 10, wherein the mixture is obtained by mixing *Lactococcus lactis* P32 KCCM13222P and *Bifidobacterium bifidum* P45 KCCM13223P at a ratio of 1:1 to 4:1 in terms of colony forming unit (CFU).

12. The pharmaceutical composition according to any one of claims 6 to 10, wherein the *Lactococcus lactis* P32 KCCM13222P or the *Bifidobacterium bifidum* P45 KCCM13223P is a live bacterium thereof, an inactivated bacterium thereof, a culture product thereof, a disrupted product thereof, or an extract thereof.

13. A food composition for prevention or amelioration of an inflammatory disease or osteoporosis, the food composition comprising:
*Lactococcus lactis* P32 KCCM13222P,
*Bifidobacterium bifidum* P45 KCCM13223P; or
a mixture thereof.

14. The food composition according to claim 13, wherein the inflammatory disease is vaginitis.

15. A food composition for prevention or amelioration of depressive disorder or anxiety disorder, the food composition comprising:
*Lactococcus lactis* P32 KCCM13222P,
*Bifidobacterium bifidum* P45 KCCM13223P; or
a mixture thereof.

16. The food composition according to claim 15, wherein the depressive disorder or the anxiety disorder is caused by an inflammatory disease or a female hormone imbalance.

17. The food composition according to any one of claims 13 to 16, wherein the mixture is obtained by mixing *Lactococcus lactis* P32 KCCM13222P and *Bifidobacterium bifidum* P45 KCCM13223P at a ratio of 1:1 to 4:1 in terms of colony forming unit (CFU).

18. The food composition according to any one of claims 13 to 16, wherein the *Lactococcus lactis* P32 KCCM13222P or the *Bifidobacterium bifidum* P45 KCCM13223P is a live bacterium thereof, an inactivated bacterium thereof, a culture product thereof, a disrupted product thereof, or an extract thereof.

19. A method for prevention or treatment of an inflammatory disease, osteoporosis, depressive disorder, or anxiety disorder, the method comprising a step of administering, to an individual, *Lactococcus lactis* P32 KCCM13222P, *Bifidobacterium bifidum* P45 KCCM13223P, or a mixture thereof.

20. Use of *Lactococcus lactis* P32 KCCM13222P, *Bifidobacterium bifidum* P45 KCCM13223P, or a mixture thereof, the use being for prevention or treatment of an inflammatory disease, osteoporosis, depressive disorder, or anxiety disorder.
